# EUROPÄISCHE PATENTSCHRIFT

(11) **EP 1 086 710 B1**
(45) Veröffentlichungstag und Bekanntmachung des Hinweises auf die Patenterteilung: **04.12.2002**
(21) Anmeldenummer: 99118883.0
(22) Anmeldetag: 24.09.1999
(51) Int. Cl.: A61L 27/38

(54) **Verfahren zur Oberflächenbeschichtung medizinischer Implantate**
Method for coating medical implants
Méthode de revêtement d'implants médicaux

(43) Veröffentlichungstag der Anmeldung: 28.03.2001
(73) Patentinhaber: Co.Don Aktiengesellschaft, 14513 Teltow (DE)
(72) Erfinder: Laube, Horst, Dr., 10115 Berlin (DE); Nickel, Erika, 10367 Berlin (DE); Matthäus, Martin, bei Scheffen, 14052 Berlin (DE); Willenbockel, Helmut, 29640 Schneverdingen (DE)
(74) Vertreter: Hansmann, Dierk, Dipl.-Ing.

(56) Entgegenhaltungen:
- WO-A-93/01843
- DE-A- 19 834 396
- DE-A- 19 915 610
- US-A- 5 441 539

## Beschreibung

Die Erfindung betrifft ein Verfahren zur Beschichtung der Oberflächen medizinischer Implantate mit lebenden Zellen mittels einer nutritiven physiologischen Flüssigkeit, wobei die Implantate in einen Aufnahmebehälter eingebracht und fixiert werden, der mit einer die Zellen enthaltenden Flüssigkeit gefüllt ist und der um wenigstens eine Achse gedreht wird.

Medizinische Implantate bzw. Prothesen werden, sofern sie nicht vom betroffenen Patienten selbst oder von dessen eineiigem Zwilling stammen, vom Empfängerorganismus als fremd eingestuft und werden deshalb vom körpereigenen Immunsystem durch sogenannte immunkompetente Zellen bekämpft. Um diese Immunreaktion zu verhindern, ist es bereits bekannt, die als Fremdkörper empfundenen Oberflächen von Implantaten bzw. Prothesen vor ihrer Verpflanzung in den menschlichen Körper vollständig mit körpereigenen oder identischen Zellen zu bedecken. Diese tragen an ihrer Oberfläche das Merkmal "eigen" und werden deshalb vom Immunsystem nicht bekämpft. Sie maskieren damit gleichsam die Fremdmerkmale des Prothesenmaterials.

Die Zellen werden üblicherweise mittels einer Zellsuspension auf das für eine Operation vorgesehene Implantat, sei es ein Organ, ein Organteil oder eine Prothese, vor der Operation aufgebracht. Dabei ist es das Ziel, das möglicht viele lebende Zellen aus der Zellsuspension auf der zu beschichtenden Oberfläche haften bleiben und auf dieser festwachsen. Die verwendeten Implantate können sowohl aus lebenden Körpern, beispielsweise aus Tierkörpern, stammen, oder aber auch aus unbelebtem biologisch unbedenklichem, d.h. inertem Material bestehen.

Nach der US-A-5441 539 ist auch eine Methode zur Beschichtung eines Implantats mit Zellen beschrieben, indem das Implantat in einem entsprechenden Kulturmedium enthaltenden Behälter um eine Achse gedreht wird. Es ist aber hiermit keine vollständige dreidimensionale Beschichtung von Implantaten/Prothesen möglich.

Aus der WO 93/01843 A1 ist in diesem Zusammenhang ein Verfahren der eingangs genannten Art bekannt geworden, das der Beschichtung zylindrischer Implantate dient. Bei diesem bekannten Verfahren werden die zu beschichtenden schlauchförmigen Implantate in eine horizontal angeordnete zylindrische Kammer eingebracht, die mit der Zellsuspension befüllt wird. Die Kammer wird anschließend entweder in eine kontinuierliche . Rotation m ihre Längsachse versetzt oder sie wird jeweils um einen vorgegebenen Winkel um diese Achse gedreht und für einen bestimmten Zeitraum in der erreichten Winkelposition gehalten, bevor sie eine neue Position gedreht und erneut gehalten wird.

Aufgabe der Erfindung ist es, ein Verfahren der eingangs genannten Art so auszubilden, daß mit seiner Hilfe die Oberflächen beliebig geformter Implantate und Prothesen biologischen oder künstlichen Ursprungs flächendeckend und vollständig mit körpereigenen oder genetisch identischen Zellen des Empfängers besiedelt werden kann.

Die Erfindung löst diese Aufgabe durch ein Verfahren mit den kennzeichnenden Merkmalen des Patentanspruchs 1. Vorteilhafte Weiterbildungen des Verfahrens nach der Erfindung sind in den weiteren Ansprüchen gegeben.

Bei dem Verfahren nach der Erfindung werden die für eine Operation vorgesehenen medizinischen Implantate dadurch mit lebenden Zellen, die in einer nutritiven physiologischen Flüssigkeit ausgesät wurden, beschichtet, daß sie in einen mit dieser Flüssigkeit gefüllten Aufnahmebehälter eingebracht werden, der anschließend derart um zwei zueinander senkrechte Drehachsen bewegt wird, daß sich die Zellen unter der Einwirkung der Schwerkraft und/oder einer durch Rotation erzeugten Zentrifugalkraft auf den zu besiedelnden Oberflächen ablagern. Bei den verwendeten Zellen kann es sich sowohl um Zellen des Empfängers handeln, die aus einem explantierten Gefäßstück gewonnen wurden, es können aber auch genetisch veränderte omnipotente Spenderzellen verwendet werden.

Die bei dem erfindungsgemäßen Verfahren verwendete Beschichtungsvorrichtung besteht aus verschiedenen modular aufgebauten Aufnahmebehältern, deren Größe jeweils dem zu beschichtenden Implantat optimal angepaßt ist, sowie aus einer Bewegungseinheit für diese Aufnahmebehälter. Letztere, auch als Beschichtungsrotator bezeichnet, ist derart ausgebildet, daß ein in diese eingebrachter Aufnahmebehälter zusammen mit einem darin angeordneten Implantat bzw. einer Prothese um zwei zueinander senkrecht stehende Achsen gleichzeitig oder getrennt voneinander in beliebigen Schritten und Geschwindigkeiten drehen läßt. Auf diese Weise wird eine absolut gleichmäßige Verteilung der verwendeten Zellen auf der zu beschichtenden Oberflächen gewährleistet.

Der Einsatz des Verfahrens nach der Erfindung trägt nachhaltig zu einer wesentlichen Erhöhung des Operationserfolges, beispielsweise bei Herzklappenoperationen, bei, indem vorzeitige Wiederholungsoperationen aufgrund von Klappenbeschädigungen durch Immunreaktionen verhindert werden. Zugleich kann die Anzahl der konventionell verabreichten Medikamente erheblich herabgesetzt werden, wodurch Anzahl und Ausmaß von Nebenwirkungen abnehmen. Damit steigt die Lebensqualität des betroffenen Patienten, während gleichzeitig die Kosten der Therapie gesenkt werden können.

Das Verfahren nach der Erfindung sieht zwei mögliche Betriebsarten der zur Durchführung dieses Verfahren verwendeten Beschichtungsvorrichtung vor: zum einen den sogenannten Steppermodus, zum anderen den Zentrifugalmodus. Beide haben ihre speziellen Charakteristika. Beim Steppermodus lassen sich durch frei wählbare Drehwinkel und Sedimentationspausen flächendeckend Besiedlungslinien auf die Oberfläche des Implantats bzw. der Prothese legen, die sich nach mehreren Umläufen zu einer flächendeckenden Besiedlung vereinigen. Beim Zentrifugalmodus andererseits wirkt anhand einer kontinuierlichen Rotation eine Zentrifugalkraft auf die Zellen, die unterhalb einer kritischen Grenze, beispielsweise etwa 500 g, die Zellen auf die Oberfläche drückt, ohne sie zu zerstören, wobei g die Erdbeschleunigung ist.

Zur Durchführung des Verfahrens nach der Erfindung wird das zu beschichtende Implantat in einen passenden Aufnahmebehälter der Beschichtungsvorrichtung eingesetzt, der gewährleistet, daß eine definierte Flüssigkeitsmenge mit definierter Zellanzahl auf die Oberflächen aufgebracht wird. Zugleich wird durch diese Anordnung erreicht, daß das zu beschichtende Implantat die automatisierten Drehungen der Beschichtungsvorrichtung exakt nachvollzieht. Schließlich wird so verhindert, daß das zu beschichtende Implantat während des Beschichtungsvorganges seine Position ändert oder in sich zusammenfällt.

Nachfolgend soll die Erfindung anhand eines in der Zeichnung dargestellten Ausführungsbeispiels näher erläutert werden. Es zeigen:
- Fig. 1: einen Aufnahmebehälter für zu beschichtende Implantate in Explosionsdarstellung,
- Fig. 2: eine Vorrichtung zur Beschichtung von Implantaten in ihrer Ausgangsstellung und
- Fig. 3: die Anordnung nach Fig. 2 in einer Momentanposition während eines Beschichtungsvorganges.

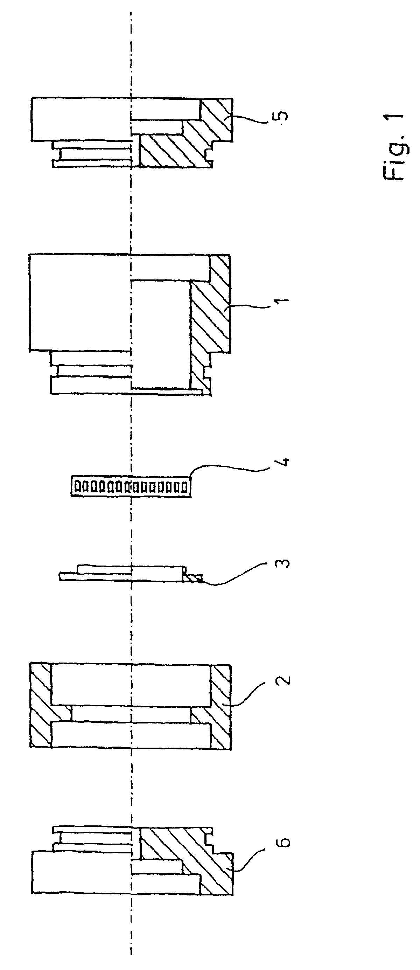

Der in Fig. 1 dargestellte Aufnahmebehälter selbst ist aus einem biologisch unbedenklichen Kunststoff gefertigt, der bei einer Temperatur von 121° C dampfsterilisierbar ist und formstabil bleibt. Er besteht aus einem Zylinder, der seinerseits aus zwei Teilen, einem Klappenträger 1 und einem Bindeglied 2 derart zusammensteckbar ist, daß zwischen diesen beiden Teilen ein Flachring 3, vorzugsweise aus einem nichtrostenden Stahl, in einer Nut gefaßt werden kann. Dieser Ring 3 trägt an seiner Unterseite eine Lippe 4, die mit regelmäßigen Bohrungen versehen ist.

Der Zylinder, der beispielsweise zur Aufnahme und Fixation einer Herzklappe dient, wird an seiner Oberund Unterseite durch zwei aufsteckbare Deckel 5,6 verschlossen, die jeweils eine zentrale Bohrung bzw. eine Fräsung aufweisen, in die ein Aufsteckfilter mit einem sogenannten Lue-Lock-Anschluß, eingesteckt wird. Diese Filter sind semipermeabel und gewährleisten somit eine Begasung, ohne die im Zylinder befindliche Flüssigkeit austreten zu lassen. Sowohl die beiden Zylinderelemente als auch die Deckelkonstruktion werden durch O-Ring-Dichtungen aus Silikonkautschuk abgedichtet, die ihrerseits bei 121° C dampfsterilisierbar sind.

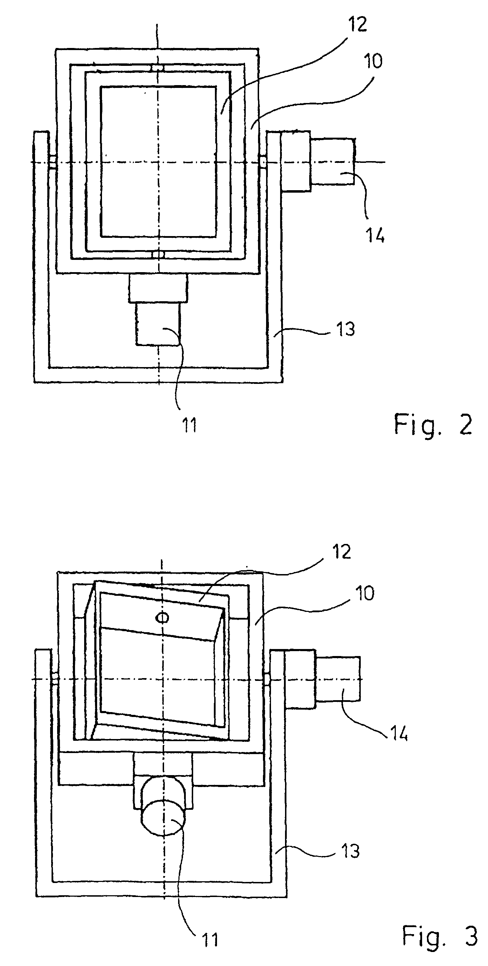

Um die Beschichtung verschiedener Implantatgrößen zu ermöglichen, werden Aufnahmebehälter mit unterschiedlichen Durchlaßdurchmessern bereitgehalten, die jeweils in den nachfolgend näher erläuterten, in den Figuren 2 und 3 dargestellten Beschichtungsrotator einbringbar sind.

Dieser Beschichtungsrotator besteht aus einer rechteckigen Zarge 10, die an einer Stirnseite in bezug auf ihre Längsachse zentriert ein Antriebsaggregat 11 trägt. Dieses Aggregat, vorzugsweise ein Schrittmotor, ist geeignet, mittels zweier Fassungen 12, die in der Längsachse der Zarge 10 fixiert werden können, den für die Beschichtung eingesetzten Aufnahmebehälter um seine Längsachse zu drehen.

Bei den Fassungen 12 handelt es sich um aus Aluminium gedrehte Kappen, die über die Deckel des Aufnahmebehälters greifen und die dort mittels eines Mitnehmers fixiert werden. Zusätzlich enthalten diese Fassungen Achsenstücke, um die der Aufnahmebehälter gedreht wird. Eines dieser Achsenstücke wird aggregatseitig mittels einer Feststellschraube in der Antriebswelle des Aggregates fixiert, das andere Achsenstück läuft auf der entgegengesetzten Seite ölgeschmiert in einer Messingführung.

Die Zarge 10 ist in einem Gestell 13 durch eine zweite Drehachse aufgehängt, welche senkrecht auf der Längsdrehachse der Zarge 10 steht. Diese zweite Drehachse wird ihrerseits durch eine Antriebseinheit 14, vorzugsweise ebenfalls einem Schrittmotor, angetrieben. Beide Antriebseinheiten 11,14 lassen sowohl Schrittdrehungen um frei wählbare Drehwinkel als auch kontinuierliche Rotationen mit frei wählbaren Geschwindigkeiten zu.

Das Gestell 13 des Beschichtungsrotators hat eine hohe Masse und ist hinreichend stabil gefertigt, um Schwingungen und Instabilitäten während des Betriebes auszugleichen. Angesteuert wird der Beschichtungsrotator über eine externe Steuerelektronik, die über einen PC zu bedienen ist

Um eine Beschichtung durchzuführen, stehen unterschiedliche Betriebsoptionen des Beschichtungsrotators bzw. dessen Steuerelektronik zur Verfügung. So ermöglicht der Steppermodus eine Beschichtung, bei der die Schwerkraft die Sedimentation der Zellen auf der zu beschichtenden Oberfläche bewirkt. Die PC-gesteuerte elektronische Steuerung dreht den eingesetzten Aufnahmebehälter von einem Startpunkt regelmäßig um einen frei wählbaren Drehwinkel. Anschließend erfolgt eine Sedimentationspause, die in ihrer Länge ebenfalls frei wählbar ist. Auch die Gesamtdauer dieses Ablaufes läßt sich frei wählen. Um zu vermeiden, daß bereits abgesiedelte Zellen durch zu starkes Beschleunigen des Aufnahmebehälters nach einer Sedimentationspause zu stark irritiert werden und sich von ihrer Unterlage lösen, ist ferner die Drehgeschwindigkeit frei wählbar.

Dadurch, daß erfindungsgemäß eine Drehung um zwei voneinander unabhängige Achsen möglich ist, ergeben sich beliebige Kombinationsmöglichkeiten für die Voreinstellung der Rotation um diese beiden Achsen. Besonders vorteilhaft ist eine Kombination, bei der eine der beiden Achsen so angesteuert wird, daß zunächst auf einer Besiedlungslinie mehrere Besiedlungspunkte angefahren werden und auf diese Weise mehrere Umläufe, jeweils mit einem gewissen Versatz, ausgeführt werden. Anschließend wird kurzzeitig die zweite Achse angesteuert, um eine nächste Besiedlungslinie einzustellen und es wiederholen sich die beschriebenen Vorgänge.

Der Zentrifugalmodus ermöglicht andererseits eine Beschichtung, bei der Zentrifugalkräfte, deren Größe abhängig von den Dimensionen des Aufnahmebehälters und seiner Fixierung in bezug auf die Drehachsen ist, auf die Zellen einwirken. Diese Zentrifugalkräfte drängen die Zellen den Fliehkräften entsprechend an die seitlichen Wände des zu beschichtenden Implantates. Auf jede Zelle der homogenen Zellsuspension wirkt bei der Rotation um eine Achse ein gerichteter Kraftvektor, der jede einzelne Zelle an einen bestimmten Ort führt. Bei einer gleichzeitigen Rotation um beide Achsen wirkt demgemäß ein resultierende Kraftvektor, der sich aus dem Vektorpaar der beiden Rotationsachsen ergibt. Die Kraftgröße sollte dabei einen bestimmten Grenzwert, beispielsweise 250 g, nicht überschreiten, um zu verhindern, daß die Zellen zerstört werden.

Eine Beschichtung von Herzklappen kann beispielsweise auf folgende Weise durchgeführt werden:
- Zunächst erfolgt eine kombinierte Drehbewegung des Aufnahmebehälters mit dem zu beschichtenden Implantat und der die Zellen enthaltenden Flüssigkeit um beide Drehachsen, wobei zuerst die Längsachse für etwa eine Minute allein angesteuert wird. Anschließend wird die Querachse dazugeschaltet. Die gesamte Laufzeit beträgt bei diesem Schritt etwa 5 Minuten.
- Die Rotation um die Längsachse des Aufnahmebehälters erfolgt in Schritten von jeweils 40 Windelgraden.
- Nach diesem Schritt wird die im Aufnahmebehälter befindliche Suspension erneut homogenisiert und es wird eine Rotation um die Querachse durchgeführt. Auch hier beträgt die Laufzeit wieder etwa 5 Minuten.
- Anschließend erfolgt eine Beschichtung der zentralen Anteile der Klappentaschen im Steppermodus. Hierzu wird eine intermittierende Bewegung um die Querachse, bei der beispielsweise drei Punkte zu je fünf Minuten Sedimentationszeit pro Besiedlungspunkt angefahren werden, durchgeführt.
- Nach einer Rotation des Aufnahmebehälters um die Längsachse um einen vorher eingestellten Winkel entsprechend der Klappenlage erfolgt eine Wiederholung des vorhergehenden Schrittes.
- Zwischen zwei Rotationsschritten wird eine Pause von zwei Minuten eingelegt.

Abschließend soll das erfindungsgemäße Verfahren anhand eines Anwendungsbeispiels, der Beschichtung einer Herzklappe der Aorten oder Pulmonalposition, näher erläutert werden. Bei dieser Herzklappe kann es sich sowohl um eine künstliche, d. h. eine Ventilklappe, als auch um eine kommerziell erhältliche biologische Klappe bzw. eine kurz zuvor aus einem Spendertier explantierte Klappe handeln.

Für die Beschichtung werden pro Quadratzentimeter der zu beschichtenden Oberfläche etwa 40000 Zellen verwendet. Als Zellenpool können entweder kommerziell erhältliche Zellinien oder aber eigens erzeugte Zellinien verwendet werden, wobei letzteren der Vorzug zu geben ist. Zu diesem Zweck wird ein etwa 5 cm langes Gefäßstück des Empfängers explantiert, beispielsweise aus der Vena saphena magna, Vena basilika oder Vena cephalika. Mit Hilfe eines Enzymes, üblicherweise Kollagenase, werden die Endothelzellen von der Kollagenmatrix gelöst und mit dem Durchströmungsmedium gewonnen. Diese Suspension wird fraktioniert und in Kulturflaschen abgefüllt. Unter Inkubationsbedingungen wird die Zellpopulation regelmäßig kontrolliert und nötigenfalls in größere Kulturflaschen umgefüllt, bis eine ausreichende Zellanzahl zur Beschichtung einer Klappe zur Verfügung steht.

Zur Vorbereitung der Beschichtung werden die Klappen gespült, beispielsweise in PBS (Phosphate buffered Saline) und dann je nach Herkunft weiterbehandelt: Die zu beschichtenden Oberflächen künstlicher Ventilklappen werden mit Fibrinkleber behandelt, um auf diese Weise eine Submatrix zu schaffen, auf der sich eine einschichtige Zellage, in diesem Fall bestehend aus Endothelzellen, etablieren kann. Kommerzielle biologische Klappen werden üblicherweise in einer Glutaraldehyd-Konservierungslösung angeliefert und werden nach dem Spülen in einem Aminosäurebad, zum Beispiel L-Glutatmin, durch eine Entfernung der Aldehydgruppen detoxifiziert. Anschließend werden die zu beschichtenden Oberflächen mit Fibronectin, einem hochmolekularen Eiweißkörper (Molekulargewicht ca. 450000), behandelt. Das Fibronectin verbindet sich sowohl mit den Zelloberflächen als auch mit interzellulären Strukturen, z. B. Kollagenfasern, auf denen die für die Beschichtung verwendeten Zellen in vivo fixiert sind. Auf diese Weise entsteht eine Submatrix, auf der eine einschichtige Lage der Endothelzellen anwachsen kann.

Aus Spendertieren explantierte, nicht vorbehandelte Klappen tragen ein eigenes spendertierspezifisches Endothel. Um dieses Entothel zu entfernen, wird nach dem Spülen zunächst eine Deendothelialisierung durchgeführt. Dies geschieht mittels eines biologischen Detergens, beispielsweise Desoxycholsäure. Nach weiterem Spülen wird wieder Fibronection benutzt, um eine einschichtige Lage aus Endothelzellen auf der Implantatoberfläche zu etablieren.

Nach erfolgter Endothelialisierung in der oben beschriebenen Weise in einem Inkubator, in dem der Beschichtungsrotator, der Aufnahmebehälter und die Klappe bei 37° C in fünfprozentigem Kohlendioxid bzw. bei 95 % Luftfeuchtigkeit gelagert werden, wird der Aufnahmebehälter aus dem Beschichtungsrotator entfernt und die restliche Zellsuspension, die nicht haftende oder tote Zellen enthält, wird abgesaugt. Der Aufnahmebehälter wird mit frischem Nährmedium aufgefüllt und erneut in den Inkubator eingebracht. Dort kann der eventuell noch lückenhafte Zellteppich bis zur vollständigen Bedeckung, der Konfluenz, heranwachsen.

Nach etwa einer Woche Inkubationszeit, wobei in regelmäßigem Abständen, etwa jeden zweiten Tag, das Medium gewechselt wird, kann die beschichtete Klappe aus dem Modul entnommen werden und steht für eine Implantation zur Verfügung.

## Patentansprüche

1. Verfahren zur Beschichtung der Oberflächen medizinischer Implantate mit lebenden Zellen mittels einer nutritiven physiologischen Flüssigkeit, wobei die Implantate in einem Aufnahmebehälter eingebracht und fixiert werden, der mit einer die Zellen enthaltenen Flüssigkeit gefüllt ist und der um wenigstens eine Achse gedreht wird, **dadurch gekennzeichnet, daß** der Aufnahmebehälter nach einem vorgebbaren Bewegungsablauf gleichzeitig und unabhängig voneinander um zwei zueinander senkrecht verlaufende Drehachsen bewegt wird.

2. Verfahren nach Anspruch 1, **dadurch gekennzeichnet, daß** die Rotationsbewegung um die Drehachsen kontinuierlich erfolgt.

3. Verfahren nach Anspruch 1, **dadurch gekennzeichnet, daß** die Rotationsbewegung um wenigstens eine der Drehachsen diskontinuierlich um vorgebbare Drehwinkel erfolgt.

4. Verfahren nach einem der Ansprüche 1 bis 3, **dadurch gekennzeichnet, daß** die Rotationsbewegungen um die beiden Drehachsen , aus Kombinationen von kontinuierlichen und diskontinuierlichen Drehbewegungen bestehen.

5. Verfahren nach einem der Ansprüche 1 bis 4, **dadurch gekennzeichnet, daß** die Rotationsbewegungen um die beiden Drehachsen über unterschiedlich lange Zeiträume durchgeführt werden.

6. Verfahren nach Anspruch 4 oder 5, **dadurch gekennzeichnet, daß** zunächst eine kontinuierliche und im Anschluß an diese eine diskontinuierliche Drehbewegung erfolgt.

7. Verfahren nach Anspruch 6, **dadurch gekennzeichnet, daß** die kontinuierliche Drehbewegung um beide Drehachsen und über einen Zeitraum von fünf Minuten erfolgt.

8. Verfahren nach einem der Ansprüche 3 bis 7, **dadurch gekennzeichnet, daß** die diskontinuierliche Rotationsbewegung um die Querachse des Aufnahmebehälters in Schritten von jeweils 120 Winkelgraden erfolgt.

9. Verfahren nach Anspruch 8, **dadurch gekennzeichnet, daß** zwischen zwei Rotationsschritten eine Pause von fünf Minuten eingelegt wird.

10. Verfahren nach einem der Ansprüche 3 bis 9, **dadurch gekennzeichnet, daß** die Rotationsbewegung um die Längsachse des Aufnahmebehälters in Schritten von jeweils 40 Winkelgraden erfolgt.

11. Verfahren nach Anspruch 10, **dadurch gekennzeichnet, daß** zwischen zwei Rotationsschritten eine Pause von zwei Minuten eingelegt wird.

12. Verfahren nach einem der Ansprüche 1 bis 9, **dadurch gekennzeichnet, daß** der Aufnahembehälter nach Beendigung der Drehbewegungen über einen vorgebbaren Zeitraum bei einer Temperatur von 37° Celsius gehalten wird.

13. Verfahren nach Anspruch 12, **dadurch gekennzeichnet, daß** der Zeitraum sieben Tage beträgt.

14. Verfahren nach Anspruch 12 oder 13, **dadurch gekennzeichnet, daß** der Aufnahmebehälter mit Kohlendioxidgas begast wird.

15. Verfahren nach einem der Ansprüche 12 bis 14, **dadurch gekennzeichnet, daß** im Aufnahmebehälter eine Luftfeuchte von 95 % aufrechterhalten wird.

## Claims

1. Method of coating the surfaces of medical implants with living cells by means of a nutritive physiological liquid, wherein the implants are introduced into and fixed in a receptacle which is filled with a liquid containing the cells and which is rotated about at least one axis, **characterized in that** the receptacle is moved about two mutually perpendicular axes of rotation, simultaneously and independently of one another, according to a preset motion sequence.

2. Method according to Claim 1, **characterized in that** the rotational movement about the axes of rotation takes place continuously.

3. Method according to Claim 1, **characterized in that** the rotational movement about at least one of the axes of rotation takes place discontinuously at preset angles of rotation.

4. Method according to one of Claims 1 to 3, **characterized in that** the rotational movements about the two axes of rotation consist of combinations of continuous and discontinuous rotational movements.

5. Method according to one of Claims 1 to 4, **characterized in that** the rotational movements about the two axes of rotation are performed over different lengths of time.

6. Method according to Claim 4 or 5, **characterized in that** the rotational movement takes place first continuously and then discontinuously.

7. Method according to Claim 6, **characterized in that** the continuous rotational movement takes place about the two axes of rotation for a period of five minutes.

8. Method according to one of Claims 3 to 7, **characterized in that** the discontinuous rotational movement about the lateral axis of the receptacle takes place in angular steps of 120 degrees each.

9. Method according to Claim 8, **characterized in that** there is a pause of five minutes between two rotation steps.

10. Method according to one of Claims 3 to 9, **characterized in that** the rotational movement about the longitudinal axis of the receptacle takes place in angular steps of 40 degrees each.

11. Method according to Claim 10, **characterized in that** there is a pause of two minutes between two rotation steps.

12. Method according to one of Claims 1 to 9, **characterized in that**, when the rotational movements have ended, the receptacle is kept at a temperature of 37° Celsius for a preset period.

13. Method according to Claim 12, **characterized in that** the period is seven days.

14. Method according to Claim 12 or 13, **characterized in that** the receptacle is flushed with carbon dioxide gas.

15. Method according to one of Claims 12 to 14, **characterized in that** an atmospheric humidity of 95% is maintained in the receptacle.

## Revendications

1. Méthode de revêtement d'implants médicaux avec des cellules vivantes et au moyen d'un liquide physiologique nutritif, les implants étant introduits et fixés dans un récipient rempli d'un liquide contenant les cellules vivantes, et tournant au moins autour d'un axe, **caractérisé en ce que** le récipient tourne simultanément autour de deux axes indépendants l'un de l'autre et perpendiculaires l'un à l'autre, selon un déroulement prédéfinissable.

2. Méthode selon la revendication 1, **caractérisée en ce que** le mouvement de rotation autour des axes est continu.

3. Méthode selon la revendication 1, **caractérisée en ce que** le mouvement de rotation autour d'au moins un des axes est discontinu suivant un écart angulaire prédéfinissable.

4. Méthode selon une des revendications 1 à 3, **caractérisée en ce que** les mouvements de rotation autour des deux axes consistent en des combinaisons de rotations continues et discontinues.

5. Méthode selon une des revendications 1 à 4, **caractérisée en ce que** les mouvements de rotation autour des deux axes sont exécutés sur des laps de temps différents.

6. Méthode selon la revendication 4 ou 5, **caractérisée en ce que** le mouvement de rotation est d'abord continu puis discontinu.

7. Méthode selon la revendication 6, **caractérisée en ce que** le récipient tourne en continu autour des deux axes, pendant cinq minutes.

8. Méthode selon une des revendications 3 à 7, **caractérisée en ce que** le mouvement de rotation discontinu autour de l'axe transversal du récipient a lieu par paliers de 120°.

9. Méthode selon la revendication 8, **caractérisée en ce que** une pause de cinq minutes est prévue entre deux étapes de rotation.

10. Méthode selon une des revendications 3 à 9, **caractérisée en ce que** le récipient tourne autour de son axe longitudinal, par paliers de 40°.

11. Méthode selon la revendication 10, **caractérisée en ce que** une pause de deux minutes est prévue entre deux étapes de rotation.

12. Méthode selon une des revendications 1 à 9, **caractérisée en ce que**, une fois les rotations terminées, le récipient est maintenu à une température de 37° Celsius pendant un laps de temps prédéfinissable.

13. Méthode selon la revendication 12, **caractérisée en ce que** le laps de temps est de sept jours.

14. Méthode selon la revendication 12 ou 13, **caractérisée en ce que** le récipient est alimenté en gaz carbonique.

15. Méthode selon une des revendications 12 à 14, **caractérisée en ce que** une humidité atmosphérique de 95 % est maintenue dans le récipient.
